# FASCICULE DE BREVET EUROPEEN

(11) **EP 4 028 042 B1**
(45) Date de publication et mention de la délivrance du brevet: **10.09.2025**
(21) Numéro de dépôt: 20775210.6
(22) Date de dépôt: 10.09.2020
(51) Int. Cl.: A61K 38/17, A61K 35/28, A61P 37/06, A61K 31/00

(54) **UTILISATION DE HDL DANS LA PROPHYLAXIE DE LA MALADIE DU GREFFON CONTRE L'HÔTE**
VERWENDUNG VON HDL ZUR PROPHYLAXE VON TRANSPLANTAT-WIRT-REAKTION
USE OF HDL IN THE PROPHYLAXIS OF GRAFT-VERSUS-HOST DISEASE

(30) Priorité: 12.09.2019 FR 1910075
(43) Date de publication de la demande: 20.07.2022
(73) Titulaire: Etablissement Français du Sang, 93210 La Plaine Saint Denis (FR); INSTITUT NATIONAL DE LA SANTE ET DE LA RECHERCHE MEDICALE - INSERM, 75013 Paris (FR); Centre Hospitalier Régional Universitaire de Besançon, 25000 Besançon (FR); Universite De Franche Comte, 25000 Besançon (FR)
(72) Inventeur: CHAGUÉ, Cécile, 25000 BESANCON (FR); DAGUINDAU, Etienne, 25770 SERRE LES SAPINS (FR); SAAS, Philippe, 25000 BESANCON (FR)
(74) Mandataire: Lavoix
(86) Numéro de dépôt international: PCT/EP2020/075306
(87) Numéro de publication internationale: WO 2021/048268

(56) Documents cités:
- EP-A1- 1 593 680
- WO-A1-2017/035405
- US-A1- 2011 256 130
- M HIDAKA ET AL: "Efficacy of bezafibrate for chronic GVHD of the liver after allogeneic hematopoietic stem cell transplantation", BONE MARROW TRANSPLANTATION, vol. 45, no. 5, 5 May 2010 (2010-05-05), GB, pages 912 - 918, XP055696329, ISSN: 0268-3369, DOI: 10.1038/bmt.2009.251

## Description

La présente invention concerne la prévention et/ou le traitement de la maladie du greffon contre l'hôte.

L'efficacité curative de la transplantation de cellules hématopoïétiques allogéniques (BMT) est considérablement diminuée par la maladie du greffe contre l'hôte (GvHD) qui entraine une mortalité et une morbidité significative.

La GvHD représente une pathologie pour laquelle la corticothérapie reste à ce jour la première ligne de traitement. Toutefois, d'une part la corticothérapie est associée à de nombreuses complications iatrogènes (diabète, perturbations du métabolisme osseux avec risque de nécroses osseuses aseptiques, hypertension artérielle, dyslipidémie, etc..), et d'autre part, le pronostic est sombre en cas de résistance à ce traitement. Il n'existe pas de consensus de traitement dans ces situations graves et les outils disponibles restent des approches d'immunosuppression interférant avec le cycle cellulaire des lymphocytes T.

Il existe donc un besoin important de traitements alternatifs de la GvHD évitant de tels effets secondaires.

La présente invention vise à répondre à ce besoin.

Les sous-groupes de lymphocytes T du donneur sont reconnus comme étant les médiateurs et effecteurs cellulaires principaux de la GvHD aiguë. Des interactions des lymphocytes T avec les cellules présentatrices d'antigènes (APC) originaires de l'hôte et du donneur sont nécessaires pour atteindre le statut de « lymphocyte T activé alloréactif » générant l'attaque cytotoxique contre les organes cibles. Cependant, le réveil préliminaire des APC par des signaux d'alarme exogènes ou endogènes provenant de cellules blessées ou « en détresse » est critique pour recruter et mener une activation des lymphocytes T efficace. Les signaux exogènes sont un groupe de profils microbiens naturels très répandus appelés « profils moléculaires associés au pathogène » (PAMP) et il est supposé qu'ils sont transloqués à partir du microbiote en cas de faiblesse des barrières physiques du corps, en particulier la muqueuse intestinale. Parmi ces PAMP, le lipopolysaccharide (LPS) est le plus fortement étudié dans la pathophysiologie de la GvHD.

Les inventeurs ont identifié une augmentation du LPS plasmatique actif libre après une allogreffe, et ont alors exploré son métabolisme afin d'identifier l'étape critique altérée pendant la transplantation de cellules hématopoïétiques allogéniques. Chez les individus sains, le LPS est généralement transporté par les HDL jusqu'au foie, où le LPS est ensuite éliminé par la bile. Plusieurs transporteurs plasmatiques sont impliqués dans le chargement du LPS sur les HDL. Les inventeurs ont ici démontré que le facteur limitant expliquant l'augmentation du LPS actif libre était une chute des HDL circulantes.

La présente invention résulte de la découverte inattendue par les inventeurs que l'administration répétée de lipoprotéines HDL neutralise les LPS dans le contexte de la GvHD, réduisant la maturation des cellules présentatrices d'antigènes et diminuant l'activation de cellules effectrices de la GvHD, les lymphocytes T Tc1, et diminuant la survenue et la gravité de la GvHD aiguë.

Ainsi, pour la première fois, le traitement proposé ici par les inventeurs, l'administration de HDL, permet de prévenir et traiter la GvHD, sans reposer sur un traitement immunosuppresseur toxique, mais sur une modulation du métabolisme lipidique sans impact sur la qualité de la reconstitution immunitaire post-greffe et avec une toxicité minime attendue sur les autres organes.

Ceci est particulièrement vrai dans le foie, où les inventeurs ont mis en évidence un infiltrat réduit de cellules T CD8⁺, ainsi qu'une activation limitée de macrophages inflammatoires résidents et non-résidents, après traitement avec des HDL dans un modèle expérimental de GvHD.

Cooke et al. (2001) J. Clin. Invest. 107 :1581 ont étudié l'utilisation d'un inhibiteur compétitif du LPS, le composé B975, dans un modèle murin de transplantation de moelle osseuse. Contrairement au composé B975, HDL n'est pas un inhibiteur compétitif du LPS mais le neutralise et l'élimine.

De plus, les HDL présentent un effet anti-inflammatoire, indépendant du LPS, renforçant encore leur intérêt pour prévenir et/ou traiter la GvHD, caractérisée sur un plan physiopathologique par une forte composante inflammatoire.

La présente invention concerne ainsi une lipoprotéine de haute densité (HDL) ou un mimétique de HDL pour son utilisation dans la prévention et/ou le traitement de la maladie du greffon contre l'hôte (GvHD) ou du syndrome de relargage des cytokines, chez un sujet, où le mimétique de HDL est : (i) une nanoparticule d'or recouverte de phospholipides et d'ApoA1 ou de mimétiques d'ApoA1, ou (ii) une HDL reconstituée à partir de mimétiques d'ApoA1.

### Description détaillée de l'invention

La présente invention est définie dans les revendications.

### HDL et mimétique de HDL

Par « lipoprotéine de haute densité » ou « HDL », on entend ici le groupe de particules de lipoprotéines le plus petit et le plus dense.

Les HDL sont bien connues de l'homme du métier et sont typiquement des lipoprotéines riches en cholestérol, phospholipides et comprenant les apolipoprotéines A1, A-II, A-IV, C-I, C-II, C-III, et E, de densité comprise entre 1,063 et 1,210 et de diamètre variant entre 5 et 12 nm.

Typiquement, les HDL utilisées dans le cadre de l'invention sont isolées de sang de donneur sain, en particulier à partir de plasma de donneur sain, par exemple par des techniques de séparation par ultracentrifugation.

Alternativement, les HDL utilisées dans le cadre de l'invention sont reconstituées à partir d'apoliproprotéine A1 purifiée ou recombinante, telle que définie ci-dessous, et de lipides sélectionnés, en particulier de phospholipides de soja.

Ainsi, des exemples de HDL pouvant être utilisés dans le cadre de l'invention incluent le CSL111 tel que décrit dans Tardif et al. (2007) JAMA 297 :1675-1682, le CSL112 tel que décrit dans Diditechenko et al. (2013) Arterioscler. Thromb. Vasc. Biol. 33 :2202-211, le CER-001 tel que décrit dans Tardif et al. (2014) Eur. Heart J. 35 :3277-3286, l'ETC-216 (aussi appelé MDCO-216, une HDL reconstituée à partir de l'ApoA1_{Milano}).

Par « mimétique de HDL », on entend ici des molécules mimant la fonction du HDL.

Des exemples de mimétique de HDL incluent des nanoparticules d'or recouvertes de phospholipides et d'ApoA1 ou de mimétiques d'ApoA1, et des HDL reconstituées à partir de mimétiques d'ApoA1 telles que la molécule ETC-642 décrite dans Di Bartolo et al. (2011) Lipids Health 10 :224.

### Applications médicales

La présente invention concerne une lipoprotéine de haute densité (HDL) telle que définie dans la section « *HDL et mimétique de HDL* » ci-dessus ou un mimétique de HDL tel que défini dans la section « *HDL et mimétique de HDL* » ci-dessus, pour son utilisation dans la prévention et/ou le traitement de la maladie du greffon contre l'hôte (GvHD) ou du syndrome de relargage des cytokines, chez un sujet.

La présente invention a également pour objet l'utilisation d'une lipoprotéine de haute densité (HDL) telle que définie dans la section « *HDL et mimétique de HDL* » ci-dessus ou d'un mimétique de HDL tel que défini dans la section « *HDL et mimétique de HDL* » ci-dessus, pour la fabrication d'un médicament destiné à la prévention et/ou au traitement de la maladie du greffon contre l'hôte (GvHD) ou du syndrome de relargage des cytokines, chez un sujet.

Un autre objet de l'invention concerne une méthode de prévention et/ou de traitement de la maladie du greffon contre l'hôte (GvHD) ou du syndrome de relargage des cytokines, comprenant l'administration d'une quantité thérapeutiquement efficace d'une lipoprotéine de haute densité (HDL) telle que définie dans la section « *HDL et mimétique de HDL* » ci-dessus ou d'un mimétique de HDL tel que défini dans la section « *HDL et mimétique de HDL* » ci-dessus, chez un sujet qui en a besoin.

Par « traitement » ou « traiter », on entend ici atteindre, partiellement ou substantiellement, un ou plusieurs des résultats suivants : réduire partiellement ou totalement l'étendue de la maladie, améliorer un symptôme clinique ou un indicateur associé à la maladie, retarder, inhiber ou prévenir la progression de la maladie, ou partiellement ou totalement retarder, inhiber ou prévenir la survenue d'une rechute de la maladie.

Dans le cadre de l'invention, le terme « prévention » ou « prévenir » se réfère à n'importe quel indice de succès pour protéger un sujet ou d'un patient (par exemple un sujet ou un patient à risque de développer une maladie) du développement, du fait de contracter ou d'avoir une maladie, y compris la prévention d'un ou plusieurs symptômes de la maladie.

Par « sujet », on entend ici un mammifère, de préférence un humain. De préférence, le sujet traité dans le cadre de l'invention souffre d'un cancer, en particulier une hémopathie maligne (telle qu'une leucémie, un lymphome ou un myélome), ou d'un trouble hématologique non-malin tel qu'un déficit immunitaire primitif, une aplasie médullaire ou une myélodysplasie.

Par « maladie du greffon contre l'hôte » ou « GvHD » ou « GvH » ou « réaction du greffon contre l'hôte », on entend ici toute réponse immunitaire à médiation par les lymphocytes T dans laquelle les lymphocytes du donneur réagissent vis-à-vis des antigènes de l'hôte, typiquement après une greffe de moelle osseuse ou allogreffe de cellules hématopoïétiques.

La GvHD est définie par son caractère « aigu » ou « chronique » selon son délai de survenue et surtout le type de manifestations cliniques qui regroupent respectivement une sémiologie inflammatoire ou fibrosante. Une forme appelée « *overlap syndrom* » caractérise une GvHD qui regroupe des caractéristiques de GvHD aiguë et chronique. La sévérité de la GvHD aiguë peut varier de légère à très grave. De façon usuelle, la sévérité de la GvHD chronique se définit quant à elle par son caractère « limité ou extensif ».

La GvHD aiguë apparaît le plus souvent au cours des 100 premiers jours qui suivent l'allogreffe. Elle affecte souvent la peau, le foie et l'intestin, mais elle peut aussi toucher d'autres organes. La GvHD aiguë peut être classée selon la gravité des symptômes :
- grade 1 : symptômes légers,
- grade 2 : symptômes modérés,
- grade 3 : symptômes graves,
- grade 4 : symptômes très graves.

Les symptômes de la GvHD aiguë incluent typiquement les symptômes suivants : sensation de brûlure et rougeur de la peau de la paume des mains ou de la plante des pieds, éruptions cutanées qui peuvent se propager au corps entier, ampoules et desquamation, anorexie, nausées, vomissements, douleurs abdominales, diarrhée associée à une malabsorption, cytolyse hépatique associée à un ictère, les anomalies hépatiques pouvant aller jusqu'à une insuffisance hépatocellulaire.

La GvHD chronique apparaît classiquement à partir du 3^{ème} mois après l'allogreffe. Elle peut durer quelques mois ou toute la vie. La GvHD chronique peut se produire juste après une GvHD aiguë ou une période sans symptômes. Les symptômes de la GvHD chronique incluent typiquement les symptômes suivants : troubles de la peau comme la sécheresse, les éruptions cutanées, les démangeaisons, la desquamation, poikilodermie, perte des propriétés élastiques de la peau aboutissant à un tableau de sclérodermie plus ou moins étendu. Un syndrome sec muqueux et/ou ophtalmique est souvent présent et traduit par une sensation de brûlure/corps étranger dans les yeux, xérostomie (sécheresse buccale) accompagnée ou non de lichen ou ulcères buccaux. Sur le plan digestif, la GvHD chronique se manifeste par une anorexie, douleurs abdominales, diarrhée, malabsorption, nausées/vomissements. De nombreux autres organes peuvent être atteints comme les poumons (fibrose pulmonaire) ou le système musculo-squelettique (polyarthrites, crampes). Il est à noter que la GvHD chronique extensive est fréquemment associée à un défaut de reconstitution immunitaire exposant le patient à des complications infectieuses opportunistes.

Dans un mode de réalisation particulier, la GvHD prévenue ou traitée dans le cadre de l'invention est la GvHD aiguë.

Dans un mode de réalisation particulièrement préféré, la GvHD prévenue ou traitée dans le cadre de l'invention est la GvHD aigüe hépatique.

Comme indiqué ci-dessus, une GvHD se produit typiquement après une greffe de cellules hématopoïétiques.

Ainsi, dans un mode de réalisation particulier, pour prévenir ou traiter la GvHD, en particulier la GvHD aiguë, le sujet à traiter a subi ou va subir une greffe, en particulier une allogreffe, de cellules hématopoïétiques.

Plus particulièrement, dans un mode de réalisation particulier visant à prévenir la GvHD, le sujet à traiter va subir une greffe, en particulier une allogreffe, de cellules hématopoïétiques.

Dans un mode de réalisation alternatif visant à traiter la GvHD, le sujet à traiter a subi une greffe, en particulier une allogreffe, de cellules hématopoïétiques.

Dans un mode de réalisation particulier, le sujet à traiter présente une rupture de la perméabilité digestive, ladite rupture pouvant typiquement être due à un traitement pré-transplantation, tel qu'une chimiothérapie et/ou une radiothérapie. Sans être lié par la théorie, ces traitements peuvent altérer l'intégrité de la barrière intestinale en induisant l'apoptose des cellules épithéliales intestinales, en promouvant l'infiltration de cellules immunitaires et en désorganisant les structures cryptes/villosités.

Par « syndrome de relargage des cytokines » ou « CRS », on entend ici une réponse inflammatoire systématique qui peut être déclenchée par différents facteurs tels que des infections et certains médicaments. Le CRS est ainsi typiquement observé comme effet secondaire après l'administration de thérapies à base d'anticorps, d'anticancéreux non-protéiques, après l'administration de lymphocytes T à récepteur antigénique chimérique (CAR-T), mais aussi immédiatement après une allogreffe de cellules hématopoïétiques ou une GvHD. Ce syndrome se caractérise typiquement par une dyspnée sévère, avec bronchospasme et hypoxie, associée à de la fièvre, des frissons, des tremblements, de l'urticaire et des angio-oedèmes, et peut conduire à une insuffisance respiratoire aigüe et au décès.

Ainsi, dans un mode de réalisation particulier visant la prévention et/ou le traitement du CRS, le sujet à traiter a été traité ou est traité avec des agents anticancéreux, en particulier des anticorps anticancéreux, ou avec des cellules CAR-T, ou a subi ou va subir une greffe de cellules hématopoïétiques.

Dans un mode de réalisation préféré visant à prévenir et/ou traiter le syndrome de relargage des cytokines, le sujet à traiter est traité ou a été traité avec des cellules CAR-T.

La lipoprotéine de haute densité (HDL) telle que définie dans la section « *HDL et mimétique de HDL* » ci-dessus ou le mimétique de HDL tel que défini dans la section « *HDL et mimétique de HDL* » ci-dessus est typiquement administré au sujet qui en a besoin dans une quantité thérapeutiquement efficace.

Par « quantité thérapeutiquement efficace », on entend ici une quantité de principe actif suffisante pour détruire, modifier, contrôler ou éliminer la maladie. Une « quantité thérapeutiquement efficace » désigne aussi une quantité de principe actif permettant de retarder ou minimiser l'étendue de la maladie. Elle se réfère également à la quantité de principe actif fournissant un bénéfice thérapeutique dans le traitement ou la prise en charge de la maladie. Enfin, l'expression « quantité thérapeutiquement efficace » signifie une quantité de principe actif, seul, ou en combinaison avec d'autres thérapies, qui fournit un bénéfice thérapeutique dans le traitement ou la prise en charge de la maladie, incluant une amélioration des symptômes associés à la maladie.

La quantité thérapeutiquement efficace dépend naturellement de l'actif considéré, du mode d'administration, de l'indication thérapeutique, de l'âge du patient et de son état.

Avantageusement, le composé actif utilisé dans le cadre de l'invention se trouve sous la forme d'une composition pharmaceutique éventuellement comprenant en outre un excipient pharmaceutiquement acceptable.

Par "pharmaceutiquement acceptable", on entend ici des compositions et entités moléculaires qui ne produisent pas de réactions secondaires, allergiques ou autrement non-désirées quand elles sont administrées à un sujet. Un excipient ou véhicule pharmaceutiquement acceptable est ainsi une matière encapsulante, un diluant, un support, ou tout autre auxiliaire de formulation liquide, semi-solide ou solide non-toxique.

Les compositions pharmaceutiques utilisées dans le cadre de l'invention sont typiquement préparées afin de s'adapter au mode d'administration. Les excipients pharmaceutiques acceptables sont typiquement déterminés en partie par la composition administrée, ainsi que par la technique particulière utilisée pour administrer la composition.

Le dosage des composés à administrer dépend du cas individuel et, comme il est bien connu de l'homme du métier, doit être adapté aux circonstances individuelles pour obtenir une quantité thérapeutique efficace et un effet optimum. Le niveau de dose thérapeutiquement efficace est spécifique pour tout patient, et dépendra en particulier d'une variété de facteurs, y compris le trouble qui est traité et la sévérité du trouble, l'activité du composé spécifique utilisé; la composition spécifique employée, l'âge, le poids corporel, la santé générale, le sexe et le régime alimentaire du patient, le temps d'administration, la voie d'administration, et le taux d'excrétion du composé spécifique utilisé, la durée du traitement, les médicaments utilisés en combinaison avec le composé spécifique employé, et des facteurs analogues bien connus du domaine médical. Par exemple, il est bien connu pour l'homme de l'art de commencer à des doses du composé à des niveaux inférieurs à ceux requis pour atteindre l'effet thérapeutique souhaité et d'augmenter progressivement la posologie jusqu'à ce que l'effet souhaité soit obtenu.

La dose quotidienne peut être administrée en une seule dose ou, en particulier lorsque de plus grandes quantités sont administrées, être divisée en plusieurs doses individuelles.

Le dosage de la substance active dépend particulièrement du mode d'administration, et est aisément déterminé par l'homme de métier. Une quantité thérapeutiquement (dose unitaire) efficace de composé peut varier de 0,01 mg/kg à 500 mg/kg, préférablement de 0,1 mg/kg à 500 mg/kg, préférablement de 0,1 mg/kg à 250 mg/kg, préférablement de 0,1 mg/kg à 100 mg/kg, préférablement de 0,1 mg/kg à 50 mg/kg, et plus préférablement de 1 mg/kg à 20 mg/kg, en une ou plusieurs administrations hebdomadaire, pendant plusieurs semaines ou mois. La dose unitaire efficace peut donc aisément être déduite d'une dose calculée pour un patient « moyen » dont le poids est de 70 kg.

Typiquement, la lipoprotéine de haute densité (HDL) telle que définie dans la section « *HDL et mimétique de HDL* » ci-dessus ou le mimétique de HDL tel que défini dans la section « *HDL et mimétique de HDL* » ci-dessus est administré à une dose de 20 mg/kg.

Dans un mode de réalisation particulier, la HDL telle que définie dans la section « *HDL et mimétique de HDL* » ci-dessus ou le mimétique de HDL tel que défini dans la section « *HDL et mimétique de HDL* » ci-dessus est administré de manière répétée, de préférence tous les deux jours.

Dans un mode de réalisation particulier, lorsque le sujet à traiter va subir une greffe de cellules hématopoïétiques, la HDL telle que définie dans la section « *HDL et mimétique de HDL* » ci-dessus ou le mimétique de HDL tel que défini dans la section « *HDL et mimétique de HDL* » ci-dessus est administré avant la greffe de cellules hématopoïétiques, de préférence pendant la phase de conditionnement, typiquement 7 jours avant la greffe de cellules hématopoïétiques.

De préférence, la HDL telle que définie dans la section « *HDL et mimétique de HDL* » ci-dessus ou le mimétique de HDL tel que défini dans la section « *HDL et mimétique de HDL* » ci-dessus est administré avant la greffe de cellules hématopoïétiques, comme défini ci-dessus, et est de nouveau administré après la greffe de cellules hématopoïétiques, typiquement 1 à 3 jours après la greffe de cellules hématopoïétiques, en particulier 1 jour après la greffe de cellules hématopoïétiques, plus particulièrement de manière répétée entre 1 et 24 jours après la greffe de cellules hématopoïétiques.

Alternativement, la HDL telle que définie dans la section « *HDL et mimétique de HDL* » ci-dessus ou le mimétique de HDL tel que défini dans la section « *HDL et mimétique de HDL* » ci-dessus commence à être administré après la greffe de cellules hématopoïétiques, typiquement 1 à 7 jours après la greffe de cellules hématopoïétiques, typiquement 1 jour après la greffe de cellules hématopoïétiques, plus particulièrement de manière répétée entre 1 et 24 jours après la greffe de cellules hématopoïétiques.

Les compositions utilisées dans le cadre de l'invention peuvent être sous forme solide, liquide ou semi-solide, adaptée aux diverses voies d'administration (orale, rectale, nasale, intra oculaire, locale (par exemple, topique, transdermique, buccale, vaginale ou sublinguale) ou parentérale (par exemple sous-cutanée, intramusculaire, intraveineuse ou intradermique)).

De préférence, la HDL ou le mimétique de HDL est administré par voie intraveineuse.

Les formulations intraveineuses contiennent la substance active dissoute, en suspension ou émulsionnée dans un véhicule stérile, éventuellement en présence d'agents émulsifiants, des stabilisants, des agents tampons et d'autres additifs classiques ; elles sont normalement réparties dans des fioles ou des flacons pour perfusion, et peuvent être stockées sous forme de produits secs à reconstituer avec de l'eau ou avec un véhicule approprié avant utilisation.

Les compositions pharmaceutiques solides peuvent être des comprimés, des gélules, des poudres, des granules, des pilules, des poudres à reconstituer etc... ; elles peuvent contenir des excipients classiques tels que des liants, des charges, des diluants, des agents de compression, des lubrifiants, des détergents, des colorants, des agents aromatisants et des agents mouillants. Les comprimés peuvent être enrobés conformément à des procédés bien connus dans le domaine technique. Des charges appropriées incluent la cellulose, le mannitol, le lactose et d'autres agents similaires.

Les compositions liquides pour administration orale peuvent être sous forme de suspensions aqueuses ou huileuses, de solutions, d'émulsions, de sirops ou d'élixirs ou peuvent être présentées sous forme de produits secs pour reconstitution avec de l'eau ou un véhicule approprié avant utilisation; Elles peuvent contenir des additifs classiques, par exemple des agents de suspension tels que le sorbitol, le sirop, la méthylcellulose, la gélatine, l'hydroxyéthylcellulose, la carboxyméthylcellulose, un gel de stéarate d'aluminium ou des matières grasses comestibles hydrogénées, des émulsifiants tels que la lécithine, le monooléate de sorbitane ou la gomme arabique; des transporteurs non aqueux (qui peuvent comprendre des huiles comestibles) tels que l'huile d'amande, l'huile de noix de coco fractionnée, des esters huileux comme des esters de glycérine, de propylène glycol ou d'alcool éthylique; des conservateurs tels que méthyle ou de propyle p-hydroxybenzoate ou l'acide sorbique et, si cela est désiré, des agents aromatisants ou colorants classiques.

Dans le cadre de la présente demande, le terme « comprenant » doit être interprété comme couvrant toutes les caractéristiques spécifiquement mentionnées, ainsi qu'éventuellement certaines non-spécifiées additionnelles. Par ailleurs, l'utilisation du terme « comprenant » décrit également le monde de réalisation dans lequel aucune caractéristique autre que celles spécifiques mentionnées n'est présente (*i.e.* « consistant en »).

La présente invention sera décrite plus en détail par les figures et exemples ci-dessous.

### Exemples

### Exemple 1

Dans cet exemple, les inventeurs montrent que l'administration intraveineuse de lymphocytes T allogéniques induit une translocation précoce du LPS ainsi qu'une neutralisation plus faible de ses propriétés inflammatoires. Ce LPS se retrouve principalement sous forme libre dans le plasma après allogreffe de cellules hématopoïétiques.

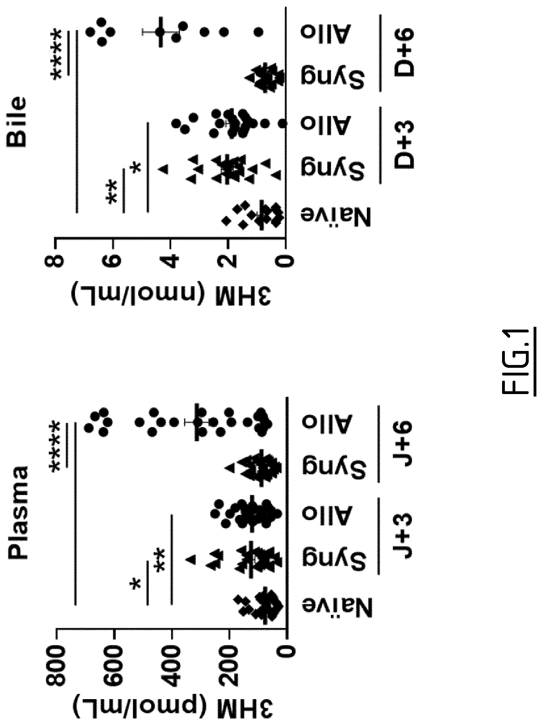

Dans un modèle murin établi de GvHD, qui consiste en des souris receveuses BALB/c irradiées de manière létale (8,5 Gy) et greffées avec 5×10⁶ moelle osseuse déplétée en lymphocytes T et 1×10⁶ lymphocytes T spléniques issus de donneuses BALB/c (syngéniques, syng) ou C57Bl/6 (allogéniques, allo), l'acide 3-hydroxymyristique (3HM) - l'acide gras hydroxylé le plus commun trouvé dans le lipide A du LPS - a été quantifié dans le plasma et la bile des souris receveuses 3 et 6 jours après la transplantation de moelle osseuse (BMT) en utilisant la technique Endoquant^{®} basée sur la chromatographie liquide à haute performance couplée à la spectrométrie de masse en tandem (Plasma : n=26-32 souris/groupe de 6 expériences indépendantes, post-test de Kruskal-Wallis et de Dunn, * : p < 0,05, ** : p < 0,01, **** : p < 0,0001 : Bile : n=10-21 souris/groupe de 3 expériences indépendantes, post-test d'ANOVA unidirectionnelle et de Bonferroni, * : p < 0,05, ** : p < 0,01, **** : p < 0,0001) (Figure 1).

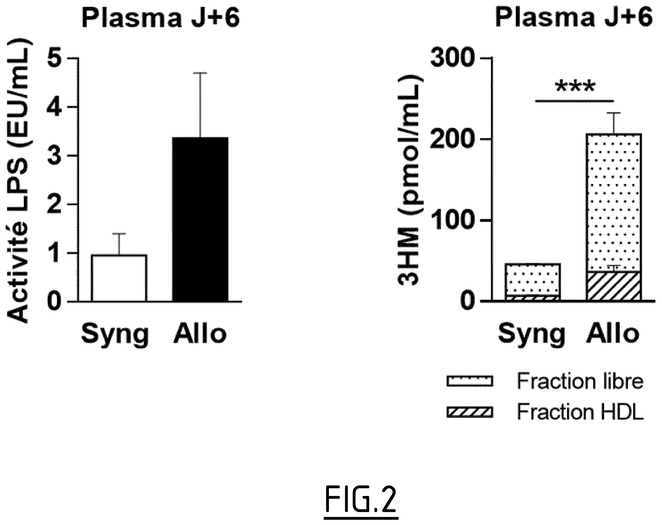

Les inventeurs ont observé que cette augmentation de LPS plasmatique après allogreffe de cellules hématopoïétiques est principalement du LPS sous sa forme libre. Les inventeurs ont confirmé l'augmentation du LPS après allogreffe à l'aide du test LAL (pour *Limulus Amoebocyte Lysate* qui repose sur la coagulation du lysat d'amébocytes contenues dans le sang de limule en présence de LPS actif, c'est-à-dire libre, uniquement) (n=9 souris/groupe de 3 expériences indépendantes) (Figure 2, gauche). Ceci a été confirmé en séparant la forme libre du LPS (non associée aux lipoprotéines circulantes) et la forme associée aux HDL par ultracentrifugation, puis quantification par la technique Endoquant^{®} (n=3-4 souris/groupe de 1 expérience, post-test d'ANOVA bidirectionnelle et de Bonferroni, *** : p < 0,001) (Figure 2, droite).

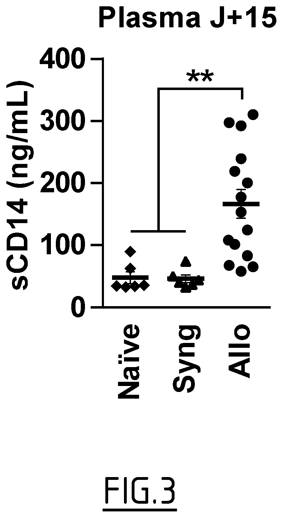

Les inventeurs ont observé que la forme soluble de CD14 (sCD14) était augmentée dans le plasma de souris allo-receveuses à j+15 après la transplantation (n=6-15 souris/groupe de 3 expériences indépendantes, post-test de Kruskal-Wallis et de Dunn, ** : p < 0,01) (Figure 3).

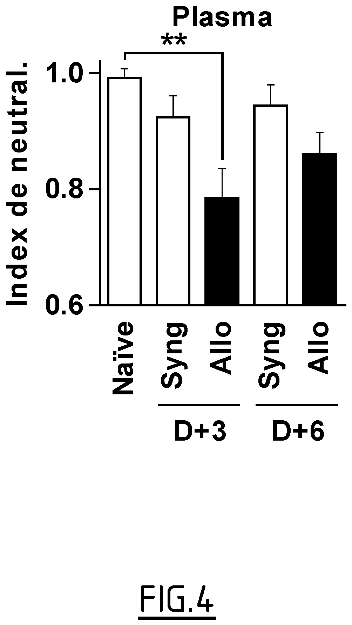

La capacité du plasma des souris receveuses à neutraliser l'activité d'une concentration connue de LPS a été étudiée *in vitro.* Brièvement, des cellules reportrices HEK-Blue TLR4/CD14/MD-2 ont été cultivées en présence de 0,5% de plasma et 0,01 EU/ml de LPS standard. Les inventeurs ont montré qu'à j+3 après la transplantation, le plasma des souris allo-receveuses présentait une activité neutralisante du LPS commercial additionné à une concentration connue (0,01 EU/mL, Invivogen) diminuée de 21,6% par rapport aux souris naïves. À j+6, la diminution était égale à 14% (n=11-16 souris/groupe de 2 expériences indépendantes, post-test d'ANOVA unidirectionnelle et de Bonferroni, ** : p < 0,01) (Figure 4).

### Exemple 2

Dans cet exemple, les inventeurs montrent que la capacité de neutralisation diminuée semble être due à un transport inverse de LPS altéré chez les souris allo-receveuses.

Le transport inverse de LPS (RLT) est une voie métabolique analogue à celle du transport inverse du cholestérol dans laquelle les substances lipophiles sont transportées au foie par des lipoprotéines (principalement HDL) afin d'être recyclées ou éliminées dans la bile.

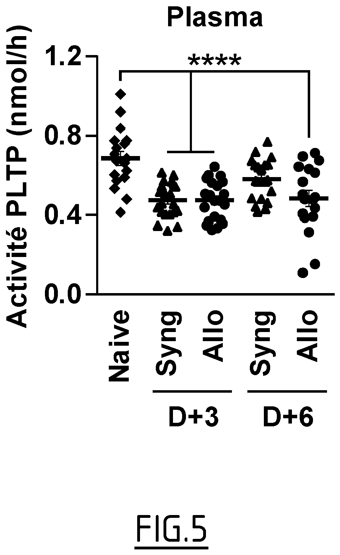

La protéine de transfert de phospholipides (PLTP) est un acteur important du RLT capable de charger le LPS sur les HDL. Les inventeurs ont montré que son activité, mesurée par un kit disponible commercialement, était légèrement diminuée par l'irradiation corporelle totale (j+3 après la transplantation) et par l'administration de lymphocytes T allogéniques (j+6) (n=18-21 souris/groupe de 3 expériences indépendantes, post-test d'ANOVA unidirectionnelle et de Bonferroni, **** : p < 0,0001) (Figure 5).

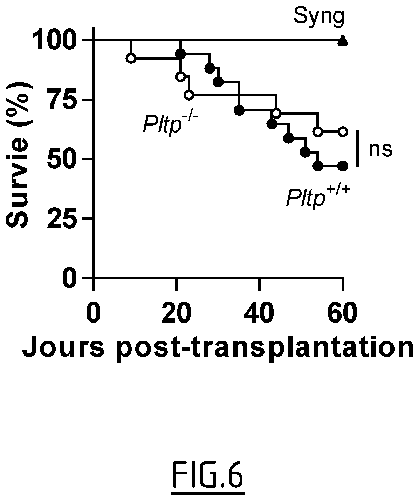

Néanmoins, les inventeurs ont mis en évidence que l'activité PLTP n'était pas l'effecteur majeur dans le contexte de la GvHD dans la mesure où son absence totale n'aggravait pas la mortalité des souris allo-greffées. Dans ce modèle, des souris C57BI/6 irradiées de manière létale (10 Gy) *Pltp*+/+ ou *Pltp*-/- ont été greffées avec 20×10⁶ moelle osseuse et 5×10⁶ lymphocytes T spléniques de donneurs C57Bl/6 (syngéniques, syng) ou C3H (allogéniques, allo) (n=12-17 souris/groupe de 2 expériences indépendantes, test de log-rank, ns : non significatif, p = 0,5338) (Figure 6).

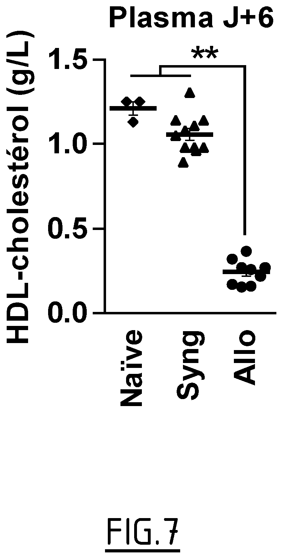

Les inventeurs ont montré que le facteur limitant principal semblait être la disponibilité des transporteurs de LPS dans la mesure où une chute précoce (j+6) de la concentration plasmatique en HDL a été observée dans deux modèles de souris GvHD. La figure 7 montre les résultats obtenus avec le modèle C57Bl/6→BALB/c (n=3-11 souris/groupe de 2 expériences indépendantes, post-test de Kruskal-Wallis et de Dunn, ** : p < 0,01).

### Exemple 3

Dans cet exemple, les inventeurs montrent que la perte de la synthèse d'apolipoprotéine A1 (ApoA1) et des HDL circulantes augmente la gravité de la GvHD.

Afin d'étudier l'effet prépondérant des HDL dans le RLT dans le contexte de la GvHD, des souris C57Bl/6 irradiées de manière létale (10 Gy) exprimant (WT) ou non (*Apoa1^{tm1Unc}*) le gène de l'ApoA1 ont été greffées avec 20×10⁶ moelle osseuse et 5×10⁶ lymphocytes T spléniques de donneurs C57Bl/6 (syngéniques, syng) ou C3H (allogéniques, allo).

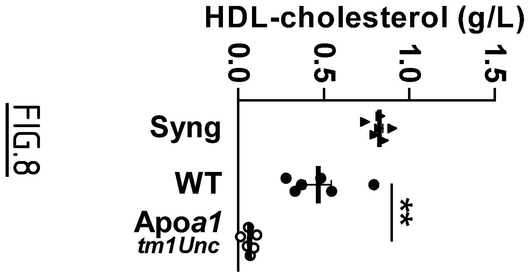

ApoA1 est l'apolipoprotéine majeure pour la formation des HDL et les inventeurs ont montré que son absence aggravait la chute des HDL dans les souris receveuses *ApoA1^{tm1Unc}* (j+6), menant à l'absence quasi-totale de HDL circulantes dans le plasma (0,067 g/l) (n=6 souris/groupe de 2 expériences indépendantes, test de Mann-Whitney, ** : p < 0,01) (Figure 8).

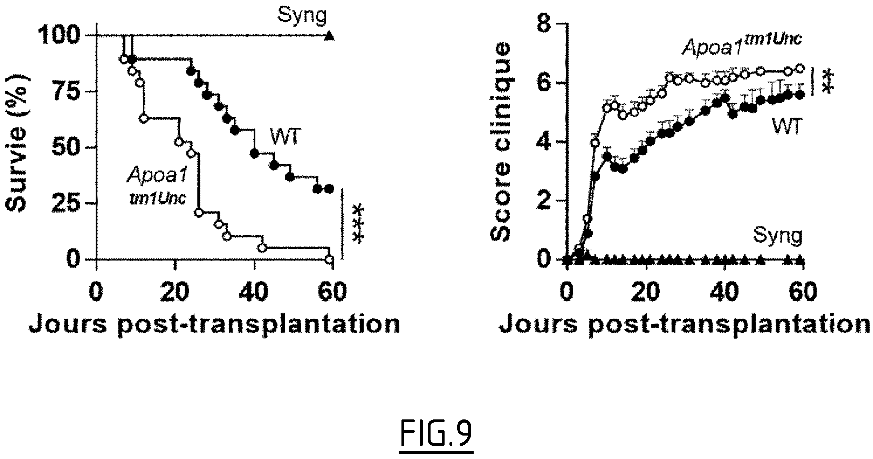

Les inventeurs ont montré que l'absence de HDL chez les souris allo-receveuses exacerbait la mortalité et la gravité de la GvHD après transplantation (n=9-19 souris/groupe de 3 expériences indépendantes, test log-rank pour la survie, *** : p < 0,001 et post-tests de Kruskal-Wallis et de Dunn sur l'ASC pour le score clinique, ** : p < 0,01) (Figure 9).

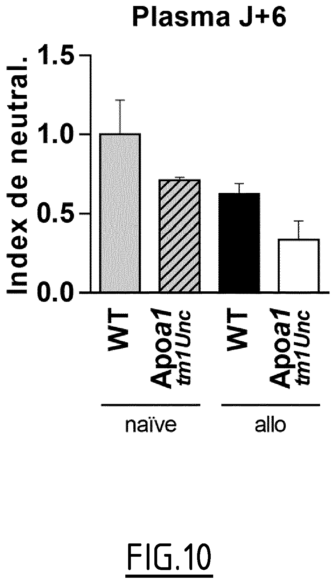

Les inventeurs ont montré que l'absence de HDL semblait être associée à une capacité altérée de neutralisation des LPS, évaluée par la méthode des cellules reportrices HEK-Blue TLR4 / CD14 / MD-2 (données préliminaires, n=3 souris/groupe de 1 expérience) (Figure 10).

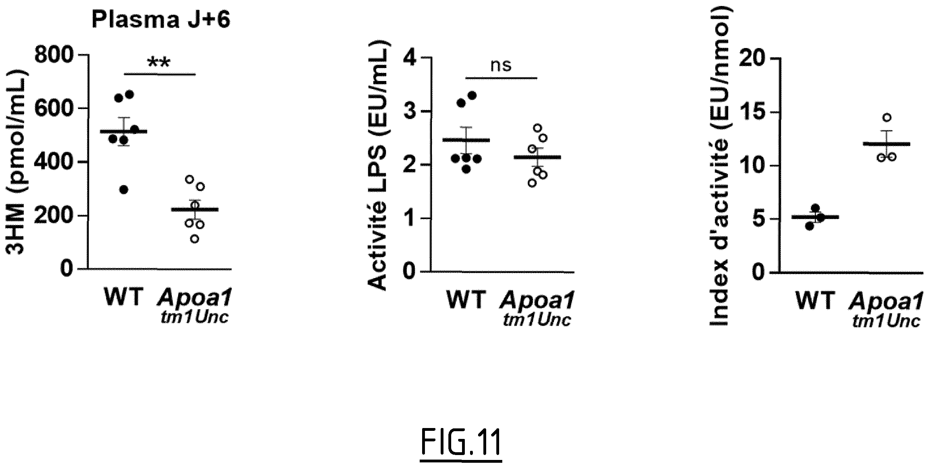

Les inventeurs ont enfin montré que les souris receveuses *ApoA1^{tm1Unc}* ont une capacité plus faible de neutralisation du LPS à j+6 après transplantation. En effet, les souris receveuses *ApoA1^{tm1Unc}* présentent des concentrations plasmatiques de 3HM (*i.e.*, LPS total) plus faibles que les souris receveuses sauvages (WT) (n=6 souris/groupe de 2 expériences indépendantes, test de Mann-Whitney, ** : p < 0,01) (Figure 11, gauche). En revanche, ces 2 groupes de souris receveuses présentent la même quantité de LPS actif appréciée par le test LAL (n=3 souris/groupe de 1 expérience) (Figue 11, centre). Cela se traduit par une capacité de neutralisation du LPS ou index d'activité plus faible chez les souris receveuses *ApoA1^{tm1Unc}* (Figure 11, droite).

### Exemple 4

Dans cet exemple, les inventeurs montrent que la perte de la synthèse d'apolipoprotéine ApoA1 et d'HDL circulant accélère la maturation des cellules dendritiques et favorise la production d'IFN-γ par les lymphocytes T dans la rate.

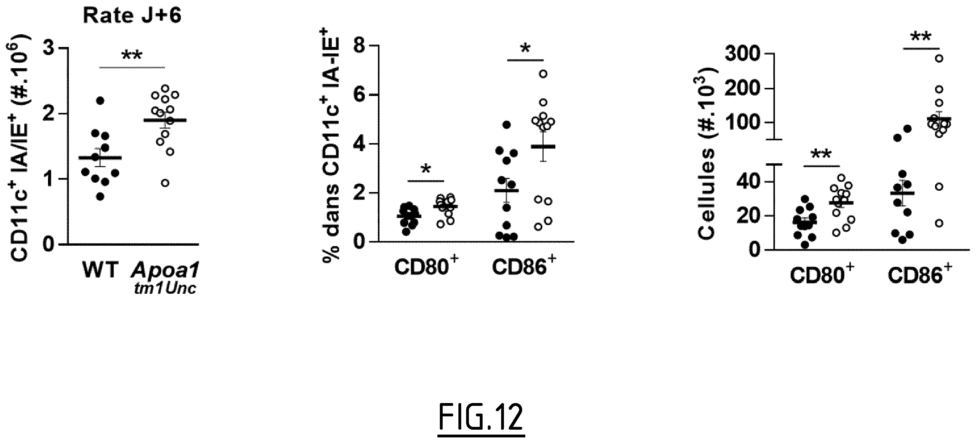

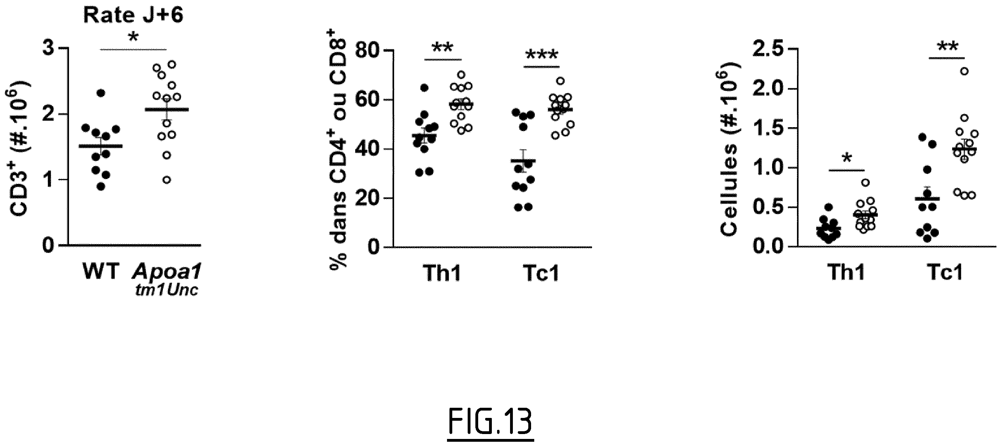

La maturation des cellules dendritiques conventionnelles (DC) (Figure 12) et la polarisation des lymphocytes T (Figure 13) ont été évaluées, par cytométrie en flux 6 jours après la transplantation, au niveau de la rate de souris C57Bl/6 irradiées de manière létale (10 Gy) exprimant (WT) ou non (*Apoa1^{tm1Unc}*) le gène de ApoA1 et greffées avec 20×10⁶ moelle osseuse et 5×10⁶ lymphocytes T spléniques provenant de donneurs C57Bl/6 (syngéniques, syng) ou C3H (allogéniques, allo).

Les inventeurs ont montré que le nombre absolu de DC CD3⁻CD19⁻CD11c⁺IA-IE⁺ vivantes était significativement augmenté dans la rate des souris receveuses *Apoa1^{tm1Unc}* (Figure 12, gauche). Ces DC présentent une expression accrue des marqueurs de maturation (CD80 et CD86) par rapport aux DC de souris allo-greffées WT en ce qui concerne le pourcentage (Figure 12, centre) et le nombre absolu (Figure 12, droite) de cellules positives (n=11-12 souris/groupe de 3 expériences indépendantes, test t non apparié ou test de Mann-Whitney, * : p < 0,05, ** : p< 0,01, *** : p < 0,001).

Les inventeurs ont également montré que le nombre absolu de lymphocytes T CD3⁺ spléniques vivants était augmenté chez les receveuses *Apoa1^{tm1Unc}* (Figure 13, gauche). Les proportions de cellules exprimant l'IFN-y parmi les cellules CD3⁺CD4⁺ (lymphocytes Th1) et les cellules CD3⁺CD8⁺ (lymphocytes Tc1) étaient plus élevées chez les receveurs *Apoa1^{tm1Unc}* (Figure 13, centre) et représentaient un nombre de cellules absolu supérieur (Figure 13, droite) après 4 heures de stimulation au phorbol-myristate-acétate/ionomycine (n = 11-12 souris/groupe de 3 expériences indépendantes, test t non apparié ou test de Mann-Whitney, * : p < 0,05, ** : p < 0,01, *** : p < 0,001).

### Exemple 5

Dans cet exemple, les inventeurs montrent que la perte de la synthèse d'apolipoprotéine ApoA1 et des HDL circulantes pourrait augmenter l'infiltration de lymphocytes T au niveau d'un organe cible de la maladie du greffon contre l'hôte, le foie, et favorise la production d'IFN-γ par ces lymphocytes T infiltrant le foie.

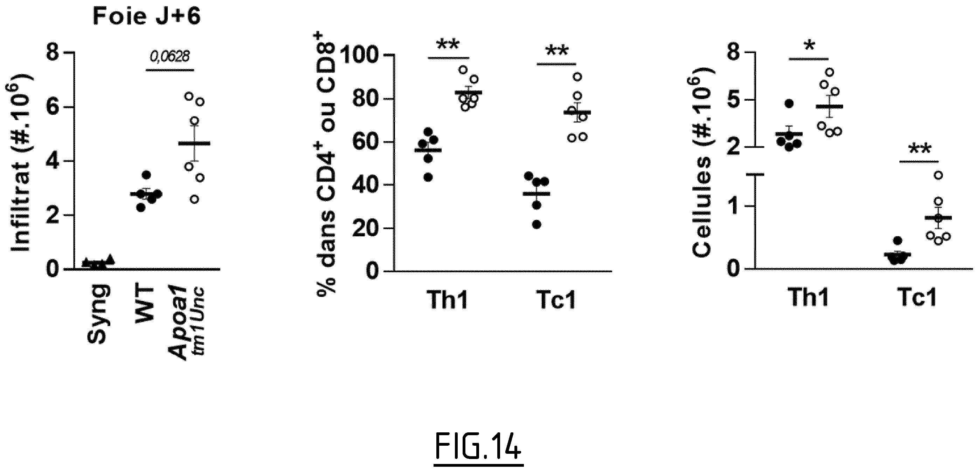

La polarisation des lymphocytes T (Figure 14) isolés du foie a été évaluée, par cytométrie en flux, 6 jours après la transplantation de souris C57Bl/6 irradiées de manière létale (10 Gy) exprimant (WT) ou non (*Apoa1^{tm1Unc}*) le gène de ApoA1 et greffées avec 20×10⁶ moelle osseuse et 5×10⁶ lymphocytes T spléniques provenant de donneurs C57Bl/6 (syngéniques, syng) ou C3H (allogéniques, allo).

Les inventeurs ont montré que le nombre absolu de lymphocytes T CD3⁺ vivants présents au niveau du foie était légèrement augmenté chez les receveuses *Apoa1^{tm1Unc}* (n=5-6 souris/groupe de 1 expérience, test de Mann-Whitney, p = 0,0628) (Figure 14, gauche). Les proportions de cellules exprimant l'IFN-y parmi les cellules CD3⁺CD4⁺ (lymphocytes Th1) et les cellules CD3⁺CD8⁺ (lymphocytes Tc1) étaient plus élevées chez les receveurs *Apoa1^{tm1Unc}* (Figure 14, centre) et représentaient un nombre de cellules absolu supérieur (Figure 14, droite) après 4 heures de stimulation au phorbol-myristate-acétate/ionomycine (n = 5-6 souris/groupe de 1 expérience, test Mann-Whitney, * : p < 0,05, ** : p < 0,01).

### Exemple 6

Dans cet exemple, les inventeurs montrent que la perte de la synthèse d'apolipoprotéine ApoA1 et des HDL circulantes résulte en une augmentation des macrophages hépatiques, principalement les macrophages non-résidents dérivés de monocytes. Ces macrophages hépatiques produisent de manière accrue des cytokines pro-inflammatoires, TNF-α et IL-6.

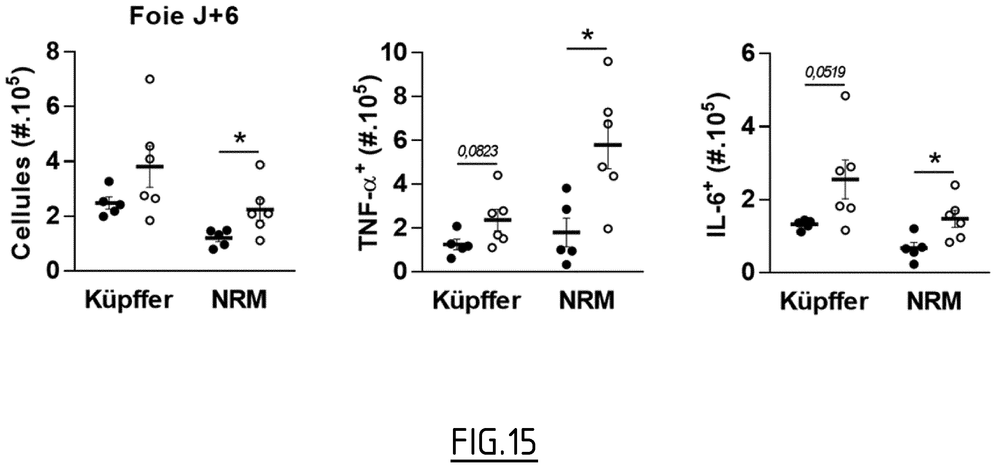

La production de cytokines par les macrophages hépatiques résidents (cellules de Küpffer) et non-résidents (NRM) (Figure 15) a été évaluée, par cytométrie en flux, 6 jours après la transplantation de souris C57Bl/6 irradiées de manière létale (10 Gy) exprimant (WT) ou non (*Apoa1^{tm1Unc}*) le gène de ApoA1 et greffées avec 20×10⁶ moelle osseuse et 5×10⁶ lymphocytes T spléniques provenant de donneurs C57Bl/6 (syngéniques, syng) ou C3H (allogéniques, allo).

Les inventeurs ont montré que le nombre absolu macrophages hépatiques résidents (cellules de Küpffer) et non-résidents (NRM) présents au niveau du foie était augmenté chez les receveuses *Apoa^{1tm1Unc}* (n=5-6 souris/groupe de 1 expérience, test de Mann-Whitney, * : p < 0,05) (Figure 15, gauche). Le nombre absolu de cellules exprimant le TNF-α (Figure 15, centre) ou l'IL-6 (Figure 15, droite) parmi les cellules F4/80^{int} CD11b^{high} (macrophages non-résidents NRM) étaient plus élevées chez les receveurs *Apoa1^{tm1Unc}* après 4 heures de stimulation par le LPS (n=5-6 souris/groupe de 1 expérience, test Mann-Whitney, * : p < 0,05). Concernant les cellules F4/80^{high} CD11b^{high} (cellules de Küpffer) exprimant ces cytokines pro-inflammatoires, il s'agit d'une tendance à l'augmentation (n=5-6 souris/groupe de 1 expérience, test Mann-Whitney, p = 0,0823 et p = 0,0519, pour le TNF-α et l'IL-6 respectivement).

### Exemple 7

Dans cet exemple, les inventeurs montrent que l'administration de HDL par voie intraveineuse (i.v.) réduit l'intensité de la GvHD et neutralise le LPS disponible.

Pour tester l'intérêt de l'utilisation prophylactique de l'administration i.v. de HDL pour modérer la gravité de la GvHD, des receveuses BALB/c irradiées de manière létale (8,5 Gy) greffés avec 5×10⁶ moelle osseuse déplétée en lymphocytes T et 1×10⁶ lymphocytes T spléniques de donneurs BALB/c (syngéniques, syng) ou C57Bl/6 (allogéniques, allo) ont été traitées par administration i.v. de HDL isolées à partir de plasma humain (20 mg/kg de poids corporel) trois fois par semaine entre j-1 et j+24 après la transplantation.

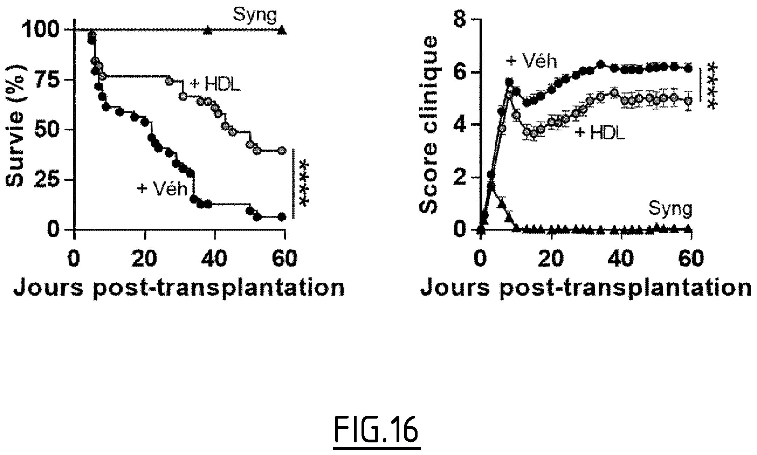

Les inventeurs ont montré que l'administration i.v. de HDL réduisait la mortalité (Figure 16, gauche) et la gravité (Figure 16, droite) de la GvHD chez les souris allo-greffées (n=19-39 souris/groupe de 4 expériences indépendantes, test log-rank pour la survie et post-test ANOVA unidirectionnelle et de Bonferroni sur l'ASC pour le score clinique, **** : p < 0,0001).

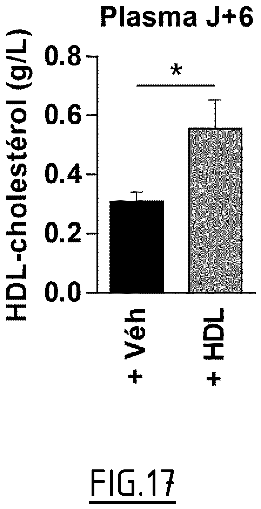

Les inventeurs ont également montré que l'administration i.v. de HDL isolées rétablissait le niveau de HDL en circulation à j+6 (n=10-11 souris/groupe de 3 expériences indépendantes, test de Mann-Whitney, * : p < 0,05) (Figure 17).

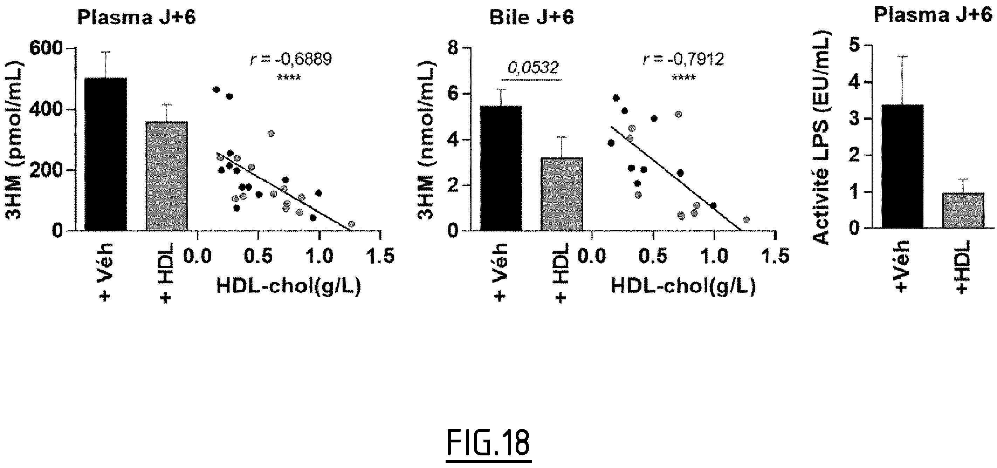

Les inventeurs ont enfin montré que l'administration i.v. de HDL semblait diminuer la concentration de 3HM dans le plasma et la bile des souris allo-greffées (n=9-13 souris/groupe de 4 expériences indépendantes, test de Mann-Whitney). Les niveaux plasmatique (Figure 18, gauche) et biliaire (Figure 18, centre) de 3HM étaient fortement corrélés à la concentration de HDL circulantes (respectivement n=13 souris/groupe de 4 expériences indépendantes, test de Mann-Whitney ; n = 26 paires, test de corrélation de Spearman non paramétrique et n=9-10 souris/groupe de 4 expériences indépendantes, test de Mann-Whitney ; n=19 paires, test de corrélation de Spearman non paramétrique, **** : p < 0,0001). Les concentrations de 3HM détectées par la technique Endoquant^{®} représentent la quantité totale de LPS présente dans les liquides biologiques. L'administration i.v. de HDL semble également diminuer l'activité endotoxinique du LPS circulant mesurée par la technique LAL (n=7-9 souris/groupe de 3 expériences indépendantes) (Figure 18, droite).

### Exemple 8

Dans cet exemple, les inventeurs présentent des données préliminaires montrant que l'administration intraveineuse (i.v.) de HDL pourrait limiter l'inflammation systémique associée à la GvHD aiguë.

Les données préliminaires obtenues par les inventeurs suggèrent qu'une administration i.v. de HDL isolées à partir de plasma humain (20 mg/kg de poids corporel) trois fois par semaine pourrait diminuer l'inflammation systémique provoquée par la GvHD aiguë chez des receveuses BALB/c irradiées de manière létale (8,5 Gy) greffées avec 5×10⁶ moelle osseuse déplétée en lymphocytes T et 1×10⁶ lymphocytes T spléniques provenant de donneurs C57Bl/6 (allo).

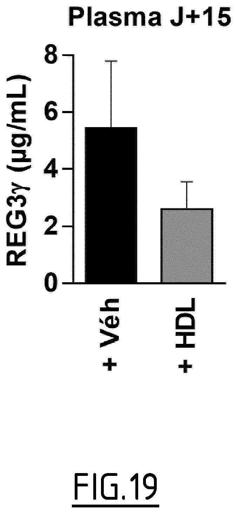

Les inventeurs ont montré que l'administration i.v. de HDL semblait limiter le niveau plasmatique du biomarqueur intestinal de GvHD aiguë REG-3γ à j+15 après la transplantation (n=8 souris/groupe de 1 expérience) (Figure 19).

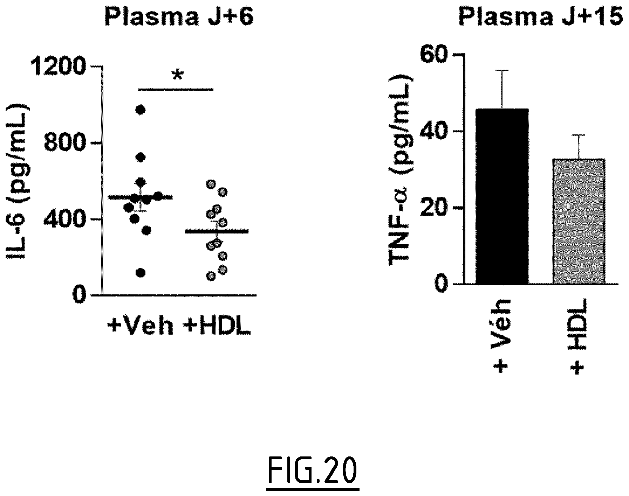

Les inventeurs ont également montré que les taux de cytokines inflammatoires circulantes avaient tendance à être diminués par l'administration i.v. de HDL : l'interleukine-6 (IL-6) était deux fois plus faible à j+6 (n=10 souris/groupe de 2 expériences indépendantes, test t non apparié, * : p < 0,05) (Figure 20, gauche) et le facteur de nécrose tumorale-α (TNF-α) est diminué de 30% à j+15 (n=3 souris/groupe de 1 expérience) (Figure 20, droite).

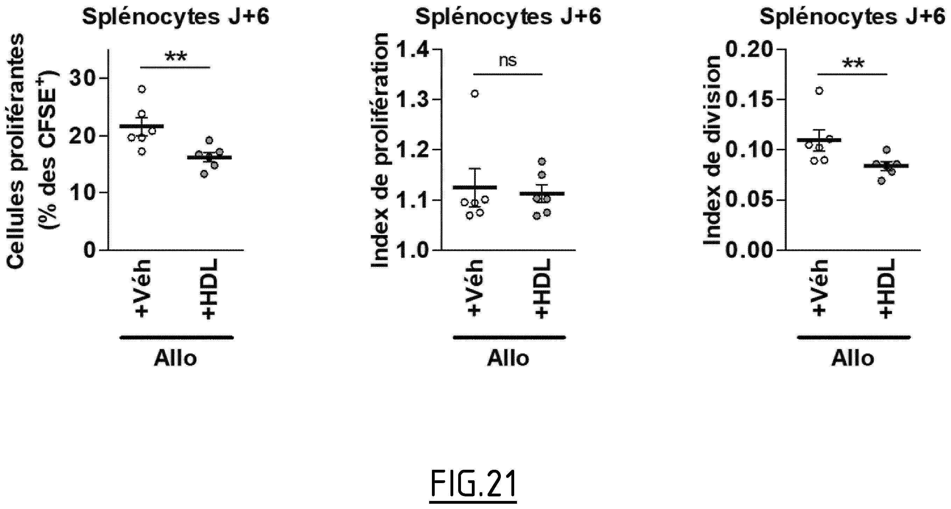

Les inventeurs ont enfin montré que les splénocytes issus de souris allogreffées traitées par administration i.v. de HDL étaient moins capables de stimuler la prolifération de lymphocytes naïfs allogéniques. Brièvement, des splénocytes issus de souris receveuses du modèle C57Bl/6 → BALB/c ont été traités à la mitomycine C et mis en culture avec des lymphocytes T issus de souris C57Bl/6 naïves et marqués au CFSE. La dilution du marquage CFSE a été analysée par cytométrie en flux après 5 jours de co-culture. La proportion de lymphocytes T ayant proliféré est significativement inférieure lorsqu'ils ont été cultivés avec des splénocytes isolés de souris traitées par administration i.v. de HDL (Figure 21, gauche). Cette plus faible proportion semble s'expliquer par un nombre inférieur de cellules rentrant en division (index de division) (Figure 21, droite) et non par une différence de vitesse de division (index de prolifération) (Figure 21, centre) (n=6 souris/groupe de 1 expérience, test de Mann-Whitney, ns : non significatif, ** : p < 0,01).

### Exemple 9

Dans cet exemple, les inventeurs présentent des données préliminaires montrant que l'administration intraveineuse (i.v.) de HDL limite la production des cytokines pro-inflammatoires TNF-α et IL-12 par les macrophages hépatiques, et l'infiltrat de lymphocytes T CD8 produisant de l'IFN-γ au niveau hépatique. Ceci se traduit par un score histologique de GvHD hépatique réduit chez les souris traitées par administration i.v. de HDL, et plus particulièrement une moindre cholangite (*i.e.*, inflammation des voies biliaires).

Les données préliminaires obtenues par les inventeurs suggèrent que l'administration i.v. de HDL isolées à partir de plasma humain (20 mg/kg de poids corporel) trois fois par semaine limite la GvHD hépatique chez des receveuses BALB/c irradiées de manière létale (8,5 Gy) greffées avec 5×10⁶ moelle osseuse déplétée en lymphocytes T et 1×10⁶ lymphocytes T spléniques provenant de donneurs C57Bl/6 (allo).

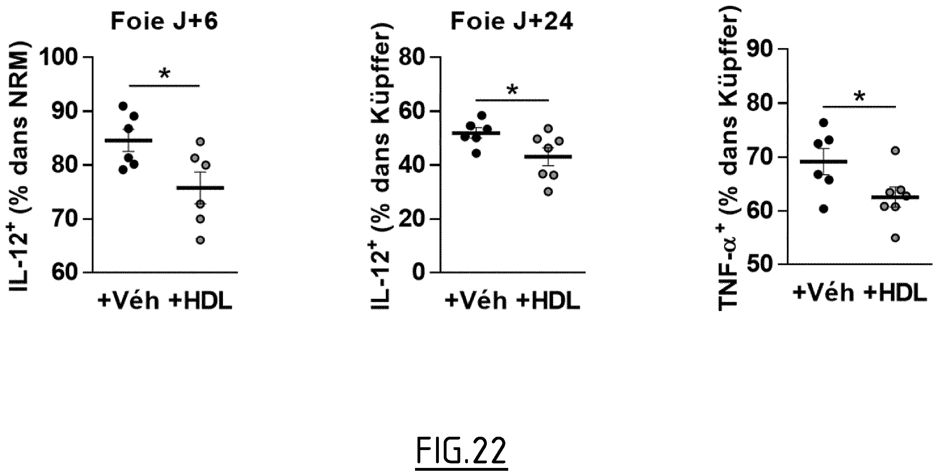

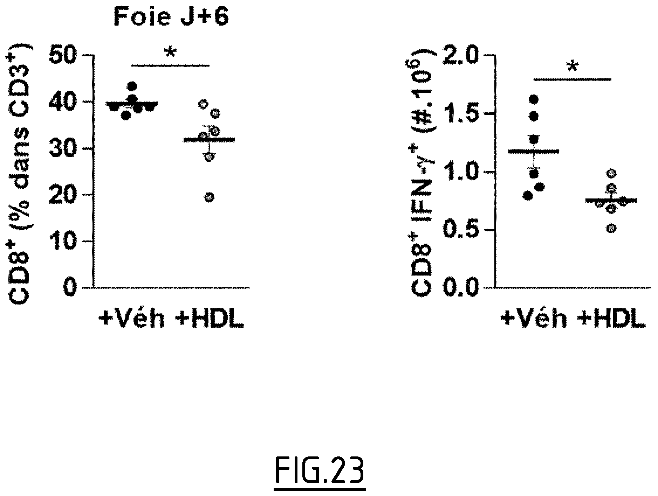

La production de cytokines par les macrophages hépatiques non-résidents (NRM) (Figure 22, gauche), résidents (cellules de Küpffer) (Figure 22, centre et droite), et les lymphocytes T CD8 (Figure 23) a été évaluée, par cytométrie en flux 6 jours ou 24 jours après la transplantation de souris BALB/c irradiées de manière létale (8,5 Gy) greffées avec 5×10⁶ moelle osseuse déplétée en lymphocytes T et 1×10⁶ lymphocytes T spléniques provenant de donneurs C57Bl/6 (allo) recevant des injections de chlorure de sodium isotonique (+ Véh) ou de HDL (+ HDL).

Les inventeurs ont montré que le pourcentage de macrophages non-résidents hépatiques (NRM) exprimant l'IL-12 était réduit chez les receveuses de HDL (+HDL) 6 jours après transplantation et après 4 heures de stimulation *in vitro* par le LPS (Figure 22, gauche) (n=6 souris/groupe de 1 expérience, test de Mann-Whitney ou test t non apparié, * : p < 0,05). Les pourcentages de macrophages hépatiques résidents (cellules de Küpffer) exprimant du TNF-α (Figure 22, droite) ou l'IL-12 (Figure 22, centre) était réduit chez les receveuses de HDL (+HDL) 24 jours après transplantation et après 4 heures de stimulation *in vitro* par le LPS (n=6 souris/groupe de 1 expérience, test de Mann-Whitney ou test non apparié, * : p < 0,05).

Les inventeurs ont aussi montré que le pourcentage de lymphocytes T CD8 au niveau hépatique des souris receveuses de HDL (+HDL) était réduit 6 jours après transplantation (Figure 23, gauche) (n=6 souris/groupe de 1 expérience, test de Mann-Whitney, * : p < 0,05). Ces lymphocytes T CD8 infiltrant le foie synthétisaient moins d'IFN-γ chez les souris receveuses de HDL (+HDL) après 4 heures de stimulation au phorbol-myristate-acétate/ionomycine. Le nombre absolu de cellules CD3⁺ CD8⁺exprimant l'IFN-γ (lymphocytes Tc1) était moins élevé chez les souris recevant des injections de HDL (+HDL) que celle recevant du chlorure de sodium isotonique (+Véh) (Figure 23, droite) (n=6 souris/groupe de 1 expérience, test de Mann-Whitney, * : p < 0,05).

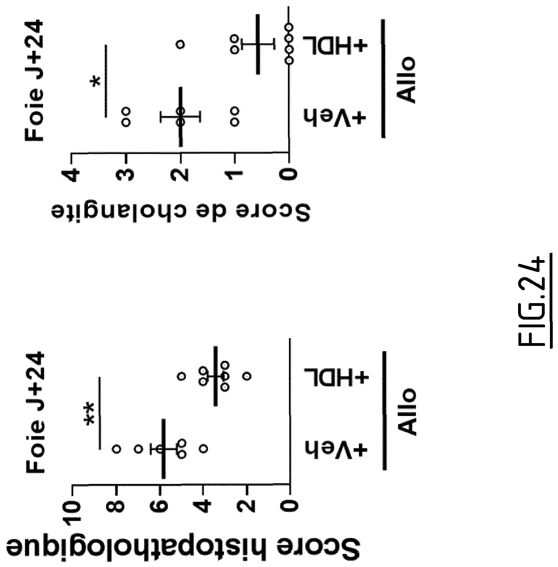

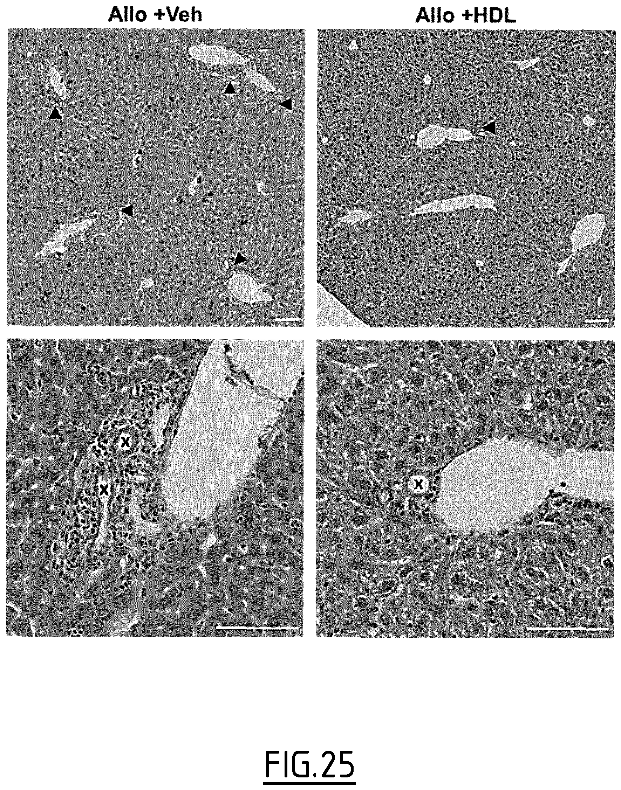

Les inventeurs ont enfin montré que le score histologique (Figure 24) - de coupes minces de foie fixées en formaline, puis inclus en paraffine et colorées par hématoxyline/éosine analysées 24 jours après la transplantation en prenant en compte les 4 critères suivants : hépatite lobulaire, infiltrat inflammatoire portal (comme montré en Figure 25, haut, les flèches indiquant l'infiltrat immunitaire dans les espaces portes et l'échelle représentant 100 µm), endothélite de la veine porte et cholangite (comme montré en Figure 25, bas, les croix indiquant les canaux biliaires et l'échelle représentant 100 µm) (chacun des paramètres évalués sur une échelle de 0 à 3, 0 étant la situation physiologique) - était réduit chez les souris traitées par administration de HDL (+HDL) en comparaison du score des souris recevant du chlorure de sodium isotonique (+Véh) (Figure 24, gauche) (n=6-7 souris/groupe de 1 expérience, test Mann-Whitney, * : p < 0,05, ** : p < 0,01). L'effet principal de l'administration i.v. de HDL était une réduction de la cholangite, c'est-à-dire l'inflammation des canaux biliaires (Figure 24, droite).

## Revendications

1. Lipoprotéine de haute densité (HDL) ou mimétique de HDL pour son utilisation dans la prévention et/ou le traitement de la maladie du greffon contre l'hôte (GvHD) ou du syndrome de relargage des cytokines, chez un sujet, où le mimétique de HDL est :
(i) une nanoparticule d'or recouverte de phospholipides et d'ApoA1 ou de mimétiques d'ApoA1, ou
(ii) une HDL reconstituée à partir de mimétiques d'ApoA1.

2. HDL ou mimétique de HDL pour son utilisation selon la revendication 1, dans laquelle la HDL est isolée de sang de donneur sain.

3. HDL ou mimétique de HDL pour son utilisation selon la revendication 1 ou 2, dans laquelle la HDL ou le mimétique de HDL est administré par voie intraveineuse.

4. HDL ou mimétique de HDL pour son utilisation selon l'une des revendications 1 à 3, dans laquelle la HDL ou le mimétique de HDL est administré de manière répétée, de préférence tous les deux jours.

5. HDL ou mimétique de HDL pour son utilisation selon l'une des revendications 1 à 4, dans laquelle la GvHD est la GvHD aiguë.

6. HDL ou mimétique de HDL pour son utilisation selon l'une des revendications 1 à 5, pour prévenir la GvHD, dans laquelle le sujet va subir une greffe de cellules hématopoïétiques.

7. HDL ou mimétique de HDL pour son utilisation selon la revendication 6, dans laquelle la HDL ou le mimétique de HDL est administré avant et après la greffe de cellules hématopoïétiques.

8. HDL ou mimétique de HDL pour son utilisation selon l'une des revendications 1 à 5, pour traiter la GvHD, dans laquelle le sujet a subi une greffe de cellules hématopoïétiques.

9. HDL ou mimétique de HDL pour son utilisation selon l'une des revendications 1 à 4, pour prévenir et/ou traiter le syndrome de relargage des cytokines, dans laquelle le sujet est traité avec des lymphocytes T à récepteur antigénique chimérique (CAR-T).

## Patentansprüche

1. Lipoprotein hoher Dichte (HDL) oder HDL-Mimetikum zur Verwendung bei der Prävention und/oder Behandlung der Graft-versus-Host-Krankheit (GvHD) oder des Zytokin-Freisetzungssyndroms bei einem Subjekt, wobei das HDL-Mimetikum wie folgt ist:
(i) ein Goldnanopartikel, beschichtet mit Phospholipiden und ApoA1 oder ApoA1-Mimetika, oder
(ii) ein HDL, rekonstituiert aus ApoA1-Mimetika rekonstituiert.

2. HDL oder HDL-Mimetikum zur Verwendung nach Anspruch 1, wobei das HDL aus dem Blut eines gesunden Spenders isoliert wird.

3. HDL oder HDL-Mimetikum zur Verwendung nach Anspruch 1 oder 2, wobei das HDL oder HDL-Mimetikum intravenös verabreicht wird.

4. HDL oder HDL-Mimetikum zur Verwendung nach einem der Ansprüche 1 bis 3, wobei das HDL oder HDL-Mimetikum wiederholt, vorzugsweise alle zwei Tage, verabreicht wird.

5. HDL oder HDL-Mimetikum zur Verwendung nach einem der Ansprüche 1 bis 4, wobei die GvHD die akute GvHD ist.

6. HDL oder HDL-Mimetikum zur Verwendung nach einem der Ansprüche 1 bis 5 zur Verhinderung der GvHD, bei der sich das Subjekt einer Transplantation hämatopoetischer Zellen unterziehen wird.

7. HDL oder HDL-Mimetikum zur Verwendung nach Anspruch 6, wobei das HDL oder HDL-Mimetikum vor und nach der Transplantation hämatopoetischer Zellen verabreicht wird.

8. HDL oder HDL-Mimetikum zur Verwendung nach einem der Ansprüche 1 bis 5 zur Behandlung von GvHD, wobei das Subjekt eine Transplantation hämatopoetischer Zellen unterzogen wurde.

9. HDL oder HDL-Mimetikum zur Verwendung nach einem der Ansprüche 1 bis 4 zur Verhinderung und/oder Behandlung des Zytokin-Freisetzungssyndroms, wobei das Subjekt mit chimären Antigenrezeptor-T-Lymphozyten (CAR-T) behandelt wird.

## Claims

1. High-density lipoprotein (HDL) or HDL mimetic for use in the prevention and/or treatment of graft-versus-host disease (GvHD) or cytokine release syndrome in a subject, wherein the HDL mimetic is:
(i) a gold nanoparticles covered in phospholipids and ApoA1 or ApoA1 mimetics, or
(ii) a HDL reconstituted from ApoA1 mimetics.

2. HDL or HDL mimetic for use according to claim 1, wherein the HDL is isolated from the blood of a healthy donor.

3. HDL or HDL mimetic for use according to claim 1 or 2, wherein the HDL or HDL mimetic is administered intravenously.

4. HDL or HDL mimetic for use according to any of claims 1 - 3, wherein the HDL or the HDL mimetic is administered repeatedly, preferably every 2 days.

5. HDL or HDL mimetic for use according to any of claims 1 - 4, wherein the GvHD is acute GvHD.

6. HDL or HDL mimetic for use according to any of claims 1 - 5 for the prevention of GvHD, wherein the subject is to undergo a haematopoietic cell transplant.

7. HDL or HDL mimetic for use according to claim 6, wherein the HDL or the HDL mimetic is administered before and after the haematopoietic cell transplant.

8. HDL or HDL mimetic for use according to any of claims 1 - 5 for the treatment of GvHD, wherein the subject has undergone a haematopoietic cell transplant.

9. HDL or HDL mimetic for use according to any of claims 1 - 4, for the prevention and/or treatment of cytokine release syndrome, wherein the subject is treated with chimeric antigen receptor T lymphocytes (CAR-T).
